# EUROPEAN PATENT SPECIFICATION

(11) **EP 3 572 822 B1**
(45) Date of publication and mention of the grant of the patent: **09.03.2022**
(21) Application number: 18173760.2
(22) Date of filing: 23.05.2018
(51) Int. Cl.: G01R 33/48, G06T 11/00, G16H 30/40, G16H 50/70, G01R 33/56, A61N 5/10

(54) **MEDICAL IMAGE CONVERSION**
UMWANDLUNG MEDIZINISCHER BILDER
CONVERSION D'IMAGES MÉDICALES

(43) Date of publication of application: 27.11.2019
(73) Proprietor: RaySearch Laboratories AB, 103 65 Stockholm (SE)
(72) Inventor: Fredriksson, Albin, 11869 Stockholm (SE); Andersson, Sebastian, 111 34 Stockholm (SE); Nilsson, Rasmus, 111 34 Stockholm (SE); Nordström, Marcus, SE-121 46 Johanneshov (SE)

(56) References cited:
- WO-A1-01/01346
- WO-A1-2018/048507
- Jelmer M. Woltering ET AL: "Deep MR to CT Synthesis using Unpaird Data", , 3 August 2017 (2017-08-03), XP055509594, Retrieved from the Internet: URL:https://pdfs.semanticscholar.org/52fc/ fc6b725220baf18cd9c70ca4ea4a3a264cca.pdf [retrieved on 2018-09-25]
- DONG NIE ET AL: "Medical Image Synthesis with Context-Aware Generative Adversarial Networks", ARXIV.ORG, CORNELL UNIVERSITY LIBRARY, 201 OLIN LIBRARY CORNELL UNIVERSITY ITHACA, NY 14853, 16 December 2016 (2016-12-16), XP080744827, DOI: 10.1007/978-3-319-66179-7_48
- AMIR M OWRANGI ET AL: "MRI-only treatment planning: benefits and challenges", PHYSICS IN MEDICINE AND BIOLOGY, INSTITUTE OF PHYSICS PUBLISHING, BRISTOL GB, vol. 63, no. 5, 26 February 2018 (2018-02-26), XP020324557, ISSN: 0031-9155, DOI: 10.1088/1361-6560/AAACA4 [retrieved on 2018-02-26]

## Description

### TECHNICAL FIELD

The present disclosure relates generally to systems and methods for generating an optimized parametrized conversion function T for converting an original medical image of a first image type into a converted medical image of a second image type.

### BACKGROUND

Computed tomography (CT) imaging is useful for many purposes. CT images may e.g. be used for dose calculation in radiation treatment planning. However, there are reasons for avoiding CT imaging if other ways of obtaining images that can be used for dose calculation can be found - CT examination takes time, and patients are exposed to radiation.

It is possible to use cone beam computed tomography (CBCT) images for dose calculation, but CBCT images are more prone to artefacts than CT images, and the intensity scale may also be different than in CT images.

The article Deep MR to CT Synthesis using Unpaired Data (Wolterink et al. 2017) describes the training of a general adversarial network (GAN) to convert MR images into CT images using a CycleGAN model and unpaired MR and CT images.

The article Medical Image Synthesis with Context-Aware Generative Adversarial Networks (Nie et al. 2016) describes the training of a fully convolutional network to convert MR images into CT images using the adversarial training strategy and an image gradient difference loss function.

### PROBLEMS WITH THE PRIOR ART

In the approach described in the article Deep MR to CT Synthesis using Unpaired Data*,* the fact that only unpaired data is used may make the generated conversion function less accurate. In this approach there is also a risk of introducing false information, which may never be detected.

In the approach described in the article Medical Image Synthesis with Context-Aware Generative Adversarial Networks*,* the fact that no comparison is made between the converted image and the original image may make the generated conversion function less accurate. This approach also requires a lot of accurate paired data.

There is thus a need for an improved method of generating an optimized parametrized conversion function T for converting an original medical image of a first image type into a converted medical image of a second image type.

### SUMMARY

The above described problem is addressed by the claimed system for generating an optimized parametrized conversion function T for converting an original medical image of a first image type into a converted medical image of a second image type. The system may comprise at least one processing unit configured to: obtain original medical images of the first and second image types; obtain an initial parametrized conversion function G to convert original medical images of the first image type into converted medical images of the second image type; calculate a first penalty P₁ based on at least one comparison of a first original medical image of the second image type with a first converted medical image of the second image type, that has been generated by applying a first parametrized conversion function G₁, which is based on the initial parametrized conversion function G, to a first original medical image of the first image type that forms an image pair with the first original medical image of the second image type and has thereby been determined to show the same part of the same patient; calculate a second penalty P₂ based on at least one comparison of an original medical image of the first image type and a converted medical image of the second image type that has been generated by applying a second parametrized conversion function G₂, which is based on the initial parametrized conversion function G, to the original medical image of the first image type, after converting the original medical image of the first image type and/or the converted medical image of the second image type into images of the same image type; and generate the optimized parametrized conversion function T based on the parameters of the initial parametrized conversion function G and at least said first and second penalties P₁ and P₂., e.g. by using estimates of the gradients of said first and second penalties P₁ and P₂, e.g. to adapt the parameters to minimize said first and second penalties P₁ and P₂. Such a system enables the generation of a very accurate optimized parametrized conversion function T.

In embodiments, the at least one processing unit is further configured to: obtain a first parametrized discriminator function D₁ to discriminate between original medical images of the second image type and converted medical images of the second image type; and calculate a third penalty P₃ based on a classification error of the first parametrized discriminator function D₁ when trying to discriminate between at least one original medical image of the second image type and at least one converted medical image of the second image type that has been generated by applying a third parametrized conversion function G₃, which is based on the initial parametrized conversion function G, to at least one original medical image of the first image type. The at least one processing unit may in this case generate the parametrized conversion function T based on the parameters of the initial parametrized conversion function G and at least said first, second and third penalties P₁, P₂ and P₃, e.g. by using estimates of the gradients of said first, second and third penalties P₁, P₂ and P₃, e.g. to adapt the parameters to minimize said first, second and third penalties P₁, P₂ and P₃. The at least one processing unit may e.g. obtain the first parametrized discriminator function D₁ based on the parameters of the parametrized discriminator function D₁ used for generating said optimized parametrized conversion function T and at least said third penalty P₃, e.g. by using estimates of the gradient of said third penalty P₃, e.g. to adapt the parameters to minimize said third penalty P₃, so that the first parametrized discriminator function D₁ is iteratively optimized. This further improves the accuracy of the optimized parametrized conversion function T.

In embodiments, the at least one processing unit is further configured to: obtain a second parametrized discriminator function D₂ to discriminate between a) image pairs with corresponding original medical images, an original medical image of the first image type and an original medical image of the second image type, which have been paired and thereby determined to show the same part of the same patient; and b) image pairs of an original medical image of the first image type and a corresponding converted medical image of the second image type; and calculate a fourth penalty P₄ based on a classification error of the second parametrized discriminator function D₂. The at least one processing unit may in this case generate the parametrized conversion function T based on the parameters of the initial parametrized conversion function G and at least said first, second and fourth penalties P₁, P₂ and P₄, e.g. by using estimates of the gradients of said first, second and fourth penalties P₁, P₂ and P₄, e.g. to adapt the parameters to minimize said first, second and fourth penalties P₁, P₂ and P₄. The at least one processing unit may e.g. obtain the second parametrized discriminator function D₂ based on the parameters of the parametrized discriminator function D₂ used for generating said optimized parametrized conversion function T and at least said fourth penalty P₄, e.g. by using estimates of the gradient of said fourth penalty P₄, e.g. to adapt the parameters to minimize said fourth penalty P₄, so that the second parametrized discriminator function D₂ is iteratively optimized. This further improves the accuracy of the optimized parametrized conversion function T.

The third and fourth penalties may of course also be combined, so that the at least one processing unit generates the parametrized conversion function T based on the parameters of the initial parametrized conversion function G and at least said first, second, third and fourth penalties P₁, P₂, P₃ and P₄, e.g. by using estimates of the gradient of said first, second, third and fourth penalties P₁, P₂, P₃ and P₄, e.g. to adapt the parameters to minimize said first, second, third and fourth penalties P₁, P₂, P₃ and P₄. This further improves the accuracy of the optimized parametrized conversion function T.

The at least one processing unit may e.g. obtain the initial parametrized conversion function G by generating it using a machine learning algorithm such as a neural network, a random forest, or a support vector machine. The machine learning algorithm may e.g. be a Generative Adversarial Network (GAN). The at least one processing unit may in further iterations obtain the initial parametrized conversion function G by using a previously generated optimized parametrized conversion function T as the initial parametrized conversion function G, so that the parametrized conversion function T is iteratively optimized.

The at least one processing unit may e.g. compare the first original medical image with the corresponding first converted medical image by voxel wise comparing the images, after first converting both images into a third image type such as e.g. a gradient image, a segmented image, or the output from a pre-trained machine learning algorithm such as a convolutional neural network, wherein the third image type amplifies certain features such as contours, regions-of-interest (ROI) and/or structures in the images so that they may more easily be compared. Alternatively, the at least one processing unit may convert the original medical image of the first image type into the second image type, or the converted medical image of the second image type into the first image type, before the comparison of the images.

The at least one processing unit may be one processing unit, or comprise several different processing units which together perform the claimed functions.

The above described problem is further addressed by the claimed processor-implemented method of generating an optimized parametrized conversion function T for converting an original medical image of a first image type into a converted medical image of a second image type. The method may comprise: obtaining original medical images of the first and second image types; obtaining an initial parametrized conversion function G to convert original medical images of the first image type into converted medical images of the second image type; calculating a first penalty P₁ based on at least one comparison of a first original medical image of the second image type with a first converted medical image of the second image type, that has been generated by applying a first parametrized conversion function G₁, which is based on the initial parametrized conversion function G, to a first original medical image of the first image type that forms an image pair with the first original medical image of the second image type and has thereby been determined to show the same part of the same patient; calculating a second penalty P₂ based on at least one comparison of an original medical image of the first image type and a converted medical image of the second image type that has been generated by applying a second parametrized conversion function G₂, which is based on the initial parametrized conversion function G, to the original medical image of the first image type, after converting the original medical image of the first image type and/or the converted medical image of the second image type into images of the same image type; and generating the optimized parametrized conversion function T based on the parameters of the initial parametrized conversion function G and at least said first and second penalties P₁ and P₂., e.g. by using estimates of the gradients of said first and second penalties P₁ and P₂, e.g. to adapt the parameters to minimize said first and second penalties P₁ and P₂. Such a method enables the generation of a very accurate optimized parametrized conversion function T.

In embodiments, the method further comprises: obtaining a first parametrized discriminator function D₁ to discriminate between original medical images of the second image type and converted medical images of the second image type; and calculating a third penalty P₃ based on a classification error of the first parametrized discriminator function D₁ when trying to discriminate between at least one original medical image of the second image type and at least one converted medical image of the second image type that has been generated by applying a third parametrized conversion function G₃, which is based on the initial parametrized conversion function G, to at least one original medical image of the first image type. The parametrized conversion function T may in this case be generated based on the parameters of the initial parametrized conversion function G and at least said first, second and third penalties P₁, P₂ and P₃, e.g. by using estimates of the gradients of said first, second and third penalties P₁, P₂ and P₃, e.g. to adapt the parameters to minimize said first, second and third penalties P₁, P₂ and P₃. The first parametrized discriminator function D₁ may e.g. be obtained based on the parameters of the parametrized discriminator function D₁ used for generating said optimized parametrized conversion function T and at least said third penalty P₃, e.g. by using estimates of the gradient of said third penalty P₃, e.g. to adapt the parameters to minimize said third penalty P₃, so that the first parametrized discriminator function D₁ is iteratively optimized. This further improves the accuracy of the optimized parametrized conversion function T.

In embodiments, the method further comprises: obtaining a second parametrized discriminator function D₂ to discriminate between a) image pairs with corresponding original medical images, an original medical image of the first image type and an original medical image of the second image type, which have been paired and thereby determined to show the same part of the same patient; and b) image pairs of an original medical image of the first image type and a corresponding converted medical image of the second image type; and calculating a fourth penalty P₄ based on a classification error of the second parametrized discriminator function D₂. The parametrized conversion function T may in this case be generated based on the parameters of the initial parametrized conversion function G and at least said first, second and fourth penalties P₁, P₂ and P₄, e.g. by using estimates of the gradients of said first, second and fourth penalties P₁, P₂ and P₄, e.g. to adapt the parameters to minimize said first, second and fourth penalties P₁, P₂ and P₄. The second parametrized discriminator function D₂ may e.g. be obtained based on the parameters of the parametrized discriminator function D₂ used for generating said optimized parametrized conversion function T and at least said fourth penalty P₄, e.g. by using estimates of the gradient of said fourth penalty P₄, e.g. to adapt the parameters to minimize said fourth penalty P₄, so that the second parametrized discriminator function D₂ is iteratively optimized. This further improves the accuracy of the optimized parametrized conversion function T.

The third and fourth penalties may of course also be combined, so that the parametrized conversion function T is generated based on the parameters of the initial parametrized conversion function G and at least said first, second, third and fourth penalties P₁, P₂, P₃ and P₄, e.g. by using estimates of the gradients of said first, second, third and fourth penalties P₁, P₂, P₃ and P₄, e.g. to adapt the parameters to minimize said first, second, third and fourth penalties P₁, P₂, P₃ and P₄. This further improves the accuracy of the optimized parametrized conversion function T.

The initial parametrized conversion function G may e.g. be obtained by being generated using a machine learning algorithm such as a neural network, a random forest, or a support vector machine. The machine learning algorithm may e.g. be a Generative Adversarial Network (GAN). In further iterations, the initial parametrized conversion function G may be obtained by a previously generated optimized parametrized conversion function T being used as the initial parametrized conversion function G, so that the parametrized conversion function T is iteratively optimized.

In embodiments, the penalties are combined in the form of a weighted sum of the penalties, so that the parameters may e.g. be adapted based on the gradient of the weighted sum of the penalties. In other embodiments, the penalties are instead applied one after the other, so that the parameters are adapted based on one penalty at a time.

The comparing of the first original medical image with the corresponding first converted medical image may e.g. voxel wise compare the images, after first converting both images into a third image type such as e.g. a gradient image, a segmented image, or the output from a pre-trained machine learning algorithm such as a convolutional neural network, wherein the third image type amplifies certain features such as contours, regions-of-interest (ROI) and/or structures in the images so that they may more easily be compared. Alternatively, the original medical image of the first image type may be converted into the second image type, or the converted medical image of the second image type may be converted into the first image type, before the comparison of the images.

The first parametrized conversion function G₁ and/or the second parametrized conversion function G₂ may e.g. be the same as the initial parametrized conversion function G. They may however also be different, e.g. by the first parametrized conversion function G₁ being based on the parameters of the initial parametrized conversion function G and the first penalty P₁, and the second parametrized conversion function G₂ being based on the parameters of either the initial parametrized conversion function G or the first parametrized conversion function G₁ and the second penalty P₂. The same may apply to the third and the fourth parametrized conversion functions G₃ and G₄. If the penalties are applied one after the other, the parametrized conversion function adapted based on one penalty may thus e.g. be used when calculating the subsequent penalty.

The image type may e.g. be a medical image modality, such as e.g. MR, CT or CBCT. In other embodiments, the image type is the output from a pre-trained machine learning algorithm such as a convolutional neural network. In other embodiments, the medical images are converted into a third image type by being segmented, by being transferred into another domain by e.g. the gradient or the frequency content in the images being calculated, or by the resolution the images being changed. In other embodiments, the first image type is a medical image of a certain medical image modality, and the second image type is an image showing the density, the stopping power, the material composition, and/or any other feature that may be used for dose calculation. In other embodiments, the first image type is a segmented image and the second image type is a medical image of a certain medical image modality. This may be useful e.g. for creating hypothetical patient images in order to simulate radiation treatment based on such hypothetical patient images.

The above described problem is further addressed by the optimized parametrized conversion function T generated by any one of the methods described above, and by the use of the optimized parametrized conversion function T generated by any one of the methods described above for converting an original medical image of a first image type into a converted medical image of a second image type, which may e.g. be used for dose calculation in radiation treatment planning, where machine instructions e.g. in the form of a control signal may be sent to radiation treatment equipment based on an analysis of the converted medical image of the second image type.

The scope of the invention is defined by the claims, which are incorporated into this section by reference. A more complete understanding of embodiments of the invention will be afforded to those skilled in the art, as well as a realization of additional advantages thereof, by a consideration of the following detailed description of one or more embodiments. Reference will be made to the appended sheets of drawings that will first be described briefly.

### BRIEF DESCRIPTION OF THE DRAWINGS

Figure 1 schematically illustrates a system for generating an optimized parametrized conversion function T for converting an original medical image of a first image type into a converted medical image of a second image type, in accordance with one or more embodiments described herein.
Figure 2 schematically illustrates a calculation of a first penalty P₁ and a second penalty P₂, in accordance with one or more embodiments described herein.
Figure 3 schematically illustrates a calculation of a third penalty P₃, in accordance with one or more embodiments described herein.
Figure 4 schematically illustrates a calculation of a fourth penalty P₄, in accordance with one or more embodiments described herein.
Figure 5 schematically illustrates a calculation of a second penalty P₂, in accordance with one or more embodiments described herein.
Figure 6 schematically illustrates a processor-implemented method of generating an optimized parametrized conversion function T for converting an original medical image of a first image type into a converted medical image of a second image type, in accordance with one or more embodiments described herein.
Figure 7 is a flow diagram exemplifying the steps of the method.

Embodiments of the present disclosure and their advantages are best understood by referring to the detailed description that follows. It should be appreciated that like reference numerals are used to identify like elements illustrated in one or more of the figures.

### DETAILED DESCRIPTION

### Introduction

As explained above, a number of different methods of training a machine learning algorithm to generate CT images based on MR images are known. However, the known methods all rely on either using paired medical images to train the algorithm, or using a feedback model, such as e.g. a CycleGAN model, to train the network using unpaired medical images. It is e.g. explained in the article Deep MR to CT Synthesis using Unpaired Data*,* which refers to the article Medical Image Synthesis with Context-Aware Generative Adversarial Networks*,* that with the use of a CycleGAN model it is not necessary to use paired medical images for training.

The present inventors have however discovered that training of the machine learning algorithm based on a combination of paired medical images and comparisons of the converted medical image with the original medical image is even more powerful, and leads to an improved machine learning algorithm. The use of paired data greatly reduces the risk of introducing false information. The machine learning algorithm may be even further improved by also being trained based on the use of discriminator functions.

Paired medical images are original medical images of different image types which have been determined to show the same part of the same patient, such as e.g. the head or any other body part of the patient. The image type may e.g. be a medical image modality, such as e.g. magnetic resonance imaging (MR) images, computed tomography (CT) images, cone beam computed tomography (CBCT) images or positron emission tomography (PET) images. In other embodiments, the image type is the output from a pre-trained machine learning algorithm such as a convolutional neural network. In other embodiments, the medical images are converted into a third image type by being segmented, by being transferred into another domain by e.g. the gradient or the frequency content in the images being calculated, or by the resolution the images being changed. In other embodiments, the first image type is a medical image of a certain medical image modality, and the second image type is an image showing the density, the stopping power, the material composition, and/or any other feature that may be used for dose calculation. In other embodiments, the first image type is a segmented image and the second image type is a medical image of a certain medical image modality.

The system and method may e.g. be used to convert images of a first image type into images of a second image type, e.g. MR images into CT images, or CBCT images into CT images, or any first image type into a second image type showing the density, the stopping power, the material composition, and/or any other feature that may be used for dose calculation. The converted images may e.g. be used for dose calculation in radiation treatment planning, and machine instructions e.g. in the form of a control signal may e.g. be sent to radiation treatment equipment based on an analysis of the converted images. The system and method may also, or alternatively, be used for removing artefacts in images.

The present disclosure relates generally to systems and methods for generating a parametrized conversion function for converting an original medical image of a first image type into a converted medical image of a second image type.

Embodiments of the disclosed solution are presented in more detail in connection with the figures.

### System architecture

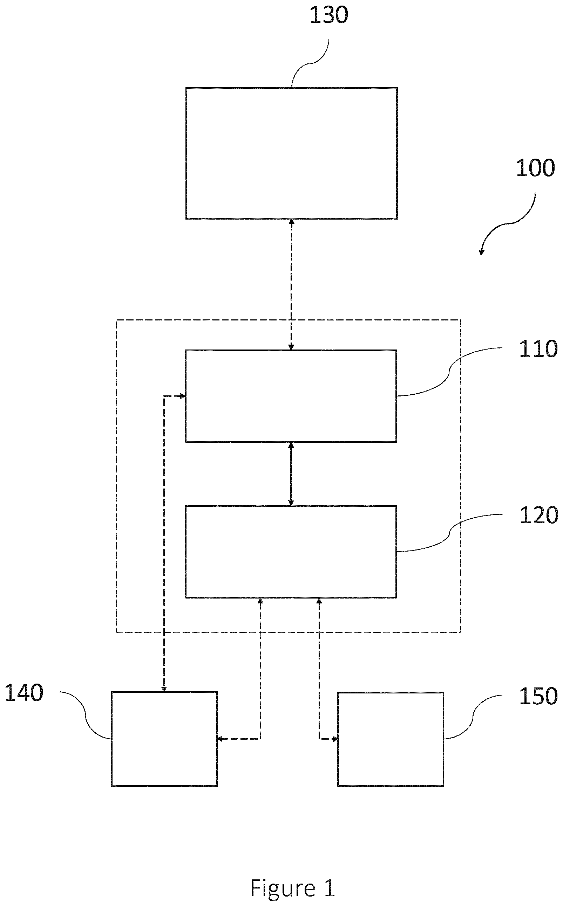

Figure 1 schematically illustrates a system 100 for generating an optimized parametrized conversion function T for converting an original medical image of a first image type into a converted medical image of a second image type. According to embodiments, the system comprises a storage media 110, configured to receive and store images and parameters. In some embodiments, the system 100 is communicatively coupled, as indicated by the dashed arrow in figure 1, to at least one imaging system 130. The imaging system 130 may be configured to capture or generate original medical images, such as for example X-ray images, ultrasound images, computed tomography (CT) images, cone beam computed tomography (CBCT) images, nuclear medicine including positron emission tomography (PET) images, and/or magnetic resonance imaging (MR) images. The storage media 110 may be configured to receive original medical images from the at least one imaging system 130 and store the received original medical images.

The system 100 further comprises at least one processing unit 120 configured to generate an optimized parametrized conversion function T for converting an original medical image of a first image type into a converted medical image of a second image type. The at least one processing unit 120 may for example be a general data processor, or other circuit or integrated circuit capable of executing instructions to perform various processing operations. The at least one processing unit 120 may be one processing unit, or comprise several different processing units which together perform the claimed functions.

In one or more embodiments, the at least one processing unit 120 is configured to: obtain original medical images of the first and second image types, e.g. from the storage media 110; obtain an initial parametrized conversion function G to convert original medical images of the first image type into converted medical images of the second image type, e.g. from the storage media 110; calculate a first penalty P₁ based on at least one comparison of a first original medical image of the second image type with a first converted medical image of the second image type, that has been generated by applying a first parametrized conversion function G₁, which is based on the initial parametrized conversion function G, to a first original medical image of the first image type that forms an image pair with the first original medical image of the second image type and has thereby been determined to show the same part of the same patient; calculate a second penalty P₂ based on at least one comparison of an original medical image of the first image type and a converted medical image of the second image type that has been generated by applying a second parametrized conversion function G₂, which is based on the initial parametrized conversion function G, to the original medical image of the first image type, after converting the original medical image of the first image type and/or the converted medical image of the second image type into images of the same image type; and generate the optimized parametrized conversion function T based on the parameters of the initial parametrized conversion function G and at least said first and second penalties P₁ and P₂, e.g. by using estimates of the gradients of said first and second penalties P₁ and P₂, e.g. to adapt the parameters to minimize said first and second penalties P₁ and P₂. The optimized parametrized conversion function T may e.g. be stored in the storage media 110.

The first penalty P₁ is thus based on paired images, by comparing the converted medical image of the second image type with the paired original medical image of the second image type. What may be compared, e.g. voxel wise, may e.g. be features such as contours, regions-of-interest (ROI) and/or structures in the images, e.g. using a pre-trained machine learning algorithm such as a convolutional neural network. If the converted medical image is very similar to the paired original medical image, the first parametrized conversion function G₁ has very accurately converted the original medical image of the first image type into a converted medical image of the second image type, and the penalty P₁ will thus be low. If the converted medical image is very different from the paired original medical image, the first parametrized conversion function G₁ has not accurately converted the original medical image of the first image type into a converted medical image of the second image type, and the penalty P₁ will thus be high. In this case, the at least one processing unit 120 may adapt the parameters of the optimized parametrized conversion function T, e.g. to minimize the penalty P₁. This may be done for any number of paired images.

The second penalty P₂ is based on comparisons, e.g. voxel wise, between converted medical images and original medical images, after converting them into images of the same image type. This may be done by either converting one of the images into an image of the other image type, or converting both images into images of a third image type. In order to voxel wise compare the images, they must however be of the same image type.

The image type does not have to be a medical image modality. In other embodiments, the image type is the output from a pre-trained machine learning algorithm such as a convolutional neural network. In other embodiments, the medical images are converted into a third image type by being segmented, by being transferred into another domain by e.g. the gradient or the frequency content in the images being calculated, or by the resolution the images being changed. In other embodiments, the first image type is a medical image of a certain medical image modality, and the second image type is an image showing the density, the stopping power, the material composition, and/or any other feature that may be used for dose calculation. In other embodiments, the first image type is a segmented image and the second image type is a medical image of a certain medical image modality. This may be useful e.g. for creating hypothetical patient images in order to simulate radiation treatment based on such hypothetical patient images.

What may be compared may e.g. be features such as contours, regions-of-interest (ROI) and/or structures in the images, e.g. using a pre-trained machine learning algorithm such as a convolutional neural network. If these properties of the two images are very similar, the second parametrized conversion function G₂ has very accurately converted the original medical image of the first image type into a converted medical image of the second image type, and the penalty P₂ will thus be low. If these properties of the two images are very different, the second parametrized conversion function G₂ has not accurately converted the original medical image of the first image type into a converted medical image of the second image type, and the penalty P₂ will thus be high. In this case, the at least one processing unit 120 may adapt the parameters of the optimized parametrized conversion function T, e.g. to minimize the penalty P₂. This may be done for any number of converted medical images.

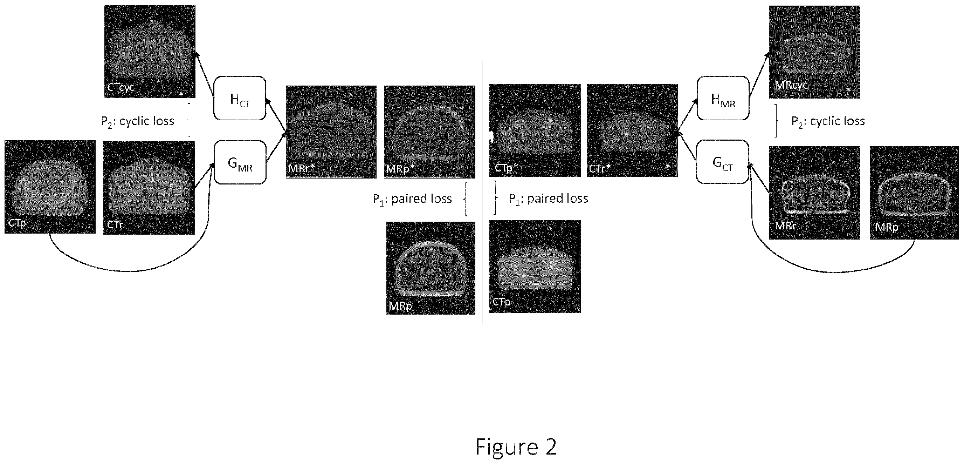

Figure 2 schematically illustrates a calculation of the first and second penalties P₁ and P₂ in a situation where a CT image is converted into an MR image (to the left in the figure) and an MR image is converted into a CT image (to the right in the figure) - each half of figure 2 is an independent process. CTp and MRp are paired original medical images, and CTr and MRr are unpaired original medical images. MRr* and MRp* are medical images which have been converted from CT images into MR images using a conversion function G_{MR}, and CTp* and CTr^{*} are medical images which have been converted from MR images into CT images using a conversion function G_{CT}. CTcyc is a medical image which has been converted from a CT image into an MR image using a conversion function G_{MR} and then converted back into a CT image using another conversion function H_{CT}, and MRcyc is a medical image which has been converted from an MR image into a CT image using a conversion function G_{CT} and then converted back into an MR image using another conversion function H_{MR}. In the embodiment described in figure 2, G_{MR} and H_{MR} are typically the same function, and G_{CT} and H_{CT} are typically the same function.

P₁ is in this illustration labelled "paired loss", since P₁ is determined based on a comparison with a paired image. P₁ may according to figure 2 be determined either by comparing MRp* with MRp, or by comparing CTp* with CTp. What may be compared, e.g. voxel wise, may e.g. be features such as contours, regions-of-interest (ROI) and/or structures in the images, e.g. using a pre-trained machine learning algorithm such as a convolutional neural network.

P₂ is in this illustration labelled cyclic loss, since P₂ may be determined based on a comparison between the original image and the converted image, after converting the converted image back into its original image type, in a cyclic manner. P₂ may in this case determine the penalty to be used for optimizing the parameters of both G and H. P₂ may according to figure 2 be determined by comparing CTr with CTcyc, which is CTr converted using a conversion function G_{MR} and converted back using another conversion function H_{CT}. P₂ may according to figure 2 also or alternatively be determined by comparing MRrwith MRcyc, which is MRr converted using a conversion function G_{CT} and converted back using another conversion function H_{MR}. The comparison may e.g. be voxel wise.

In one or more embodiments, the at least one processing unit 120 is further configured to: obtain a first parametrized discriminator function D₁ to discriminate between original medical images of the second image type and converted medical images of the second image type; and calculate a third penalty P₃ based on a classification error of the first parametrized discriminator function D₁ when trying to discriminate between at least one original medical image of the second image type and at least one converted medical image of the second image type that has been generated by applying a third parametrized conversion function G₃, which is based on the initial parametrized conversion function G, to at least one original medical image of the first image type.

The at least one processing unit 120 may in this case generate the parametrized conversion function T based on the parameters of the initial parametrized conversion function G and at least said first, second and third penalties P₁, P₂ and P₃, e.g. by using estimates of the gradients of said first, second and third penalties P₁, P₂ and P₃, e.g. to adapt the parameters to minimize said first, second and third penalties P₁, P₂ and P₃. The at least one processing unit may e.g. obtain the first parametrized discriminator function D₁ based on the parameters of the parametrized discriminator function D₁ used for generating said optimized parametrized conversion function T and at least said third penalty P₃, e.g. by using estimates of the gradient of said third penalty P₃, e.g. to adapt the parameters to minimize said third penalty P₃, so that the first parametrized discriminator function D₁ is iteratively optimized.

The third penalty P₃ is thus based on a classification error of a discriminator function when trying to discriminate between original and converted medical images of the same image type. The original medical image used may be any original medical image, it does not have to be paired with the original medical image on which the converted medical image is based. Such discriminator functions are often used for the training of adversarial networks. The conversion function tries to produce converted images that are realistic enough to fool the discriminator function, and the discriminator function tries to distinguish original and converted images. One way of doing this in a GAN framework is described in the article Medical Image Synthesis with Context-Aware Generative Adversarial Networks*.* The discriminator function is preferably iteratively trained based on at least the classification error P₃, e.g. by using estimates of the gradient of the classification error P₃, e.g. to adapt the parameters to minimize said third penalty P₃.

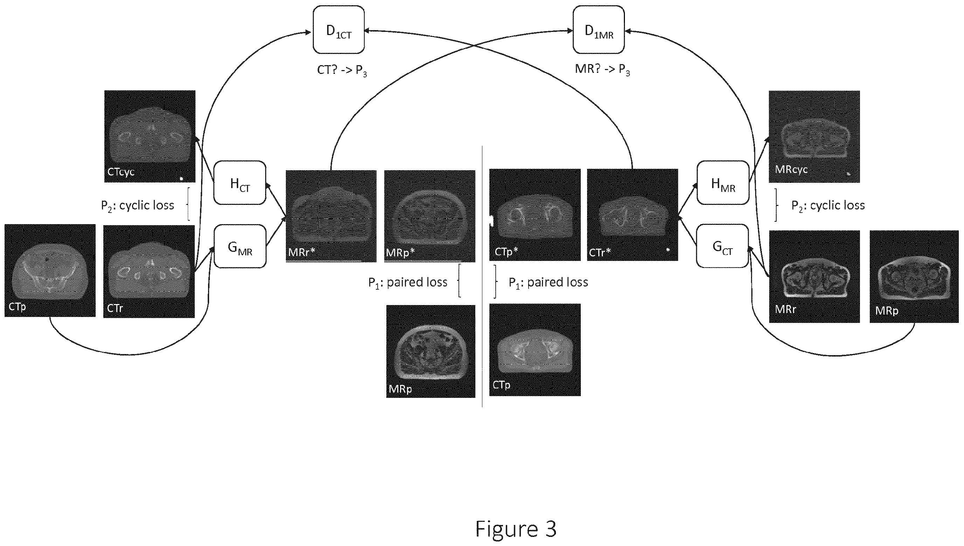

Figure 3 schematically illustrates a calculation of the third penalty P₃. P₃ may according to figure 3 be determined based on a classification error of a first discriminator function D_{1CT} when trying to distinguish between CTr and CTr*, and/or a classification error of a first discriminator function D_{1MR} when trying to distinguish between MRr and MRr*. The classification error may e.g. be determined as a number between 0 and 1, representing the percentage or likelihood of a classification error.

In one or more embodiments, the at least one processing unit 120 is further configured to: obtain a second parametrized discriminator function D₂ to discriminate between a) image pairs with corresponding original medical images, an original medical image of the first image type and an original medical image of the second image type, which have been paired and thereby determined to show the same part of the same patient; and b) image pairs of an original medical image of the first image type and a corresponding converted medical image of the second image type; and calculate a fourth penalty P₄ based on a classification error of the second parametrized discriminator function D₂. The at least one processing unit 120 may in this case generate the parametrized conversion function T based on the parameters of the initial parametrized conversion function G and of at least said first, second and fourth penalties P₁, P₂ and P₄, e.g. by using estimates of the gradients of said first, second and fourth penalties P₁, P₂ and P₄, e.g. to adapt the parameters to minimize said first, second and fourth penalties P₁, P₂ and P₄. The at least one processing unit may e.g. obtain the second parametrized discriminator function D₂ based on the parameters of the parametrized discriminator function D₂ used for generating said optimized parametrized conversion function T and at least said fourth penalty P₄, e.g. by using estimates of the gradient of said fourth penalty P₄, e.g. to adapt the parameters to minimize said fourth penalty P₄, so that the second parametrized discriminator function D₂ is iteratively optimized.

The fourth penalty P₄ is thus based on a classification error of a discriminator function when trying to discriminate between pairs of original paired medical images and pairs of original and corresponding converted medical images.

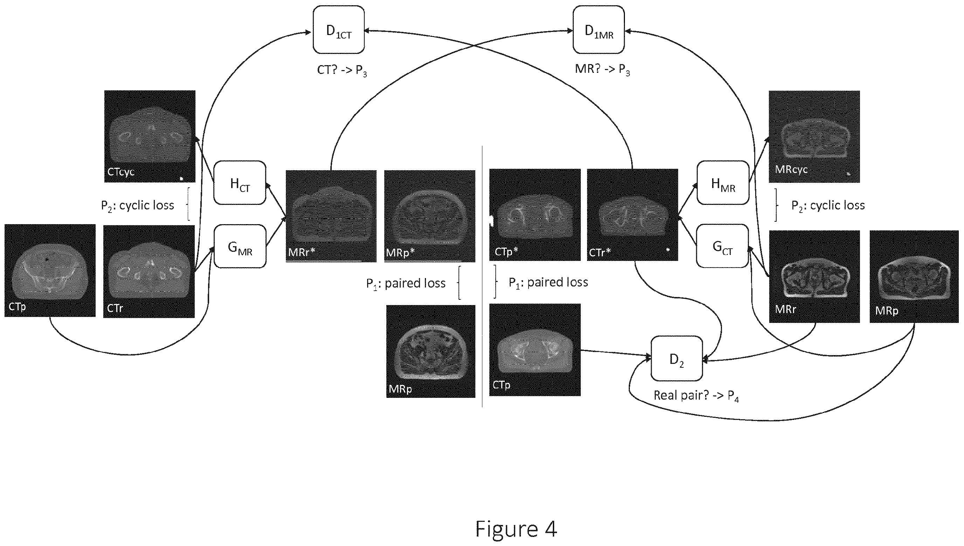

Figure 4 schematically illustrates a calculation of the fourth penalty P₄. P₄ may according to figure 4 be determined based on a classification error of the second discriminator function D₂ when trying to distinguish between the image pair CTp and MRp and the image pair CTr^{*} and MRr. A corresponding penalty P₄ may of course also be calculated in the process to the left in figure 4. The classification error may e.g. be determined as a number between 0 and 1, representing the percentage or likelihood of a classification error.

The third and fourth penalties may of course also be combined, so that the at least one processing unit generates the parametrized conversion function T based on the parameters of the initial parametrized conversion function G and at least said first, second, third and fourth penalties P₁, P₂, P₃ and P₄, e.g. by using estimates of the gradients of said first, second, third and fourth penalties P₁, P₂, P₃ and P₄, e.g. to adapt the parameters to minimize said first, second, third and fourth penalties P₁, P₂ P₃ and P₄. This further improves the accuracy of the optimized parametrized conversion function T.

In embodiments, the penalties are combined in the form of a weighted sum of the penalties, so that the parameters are adapted e.g. based on the gradient of the weighted sum of the penalties. In other embodiments, the penalties are instead applied one after the other, so that the parameters are adapted based on one penalty at a time.

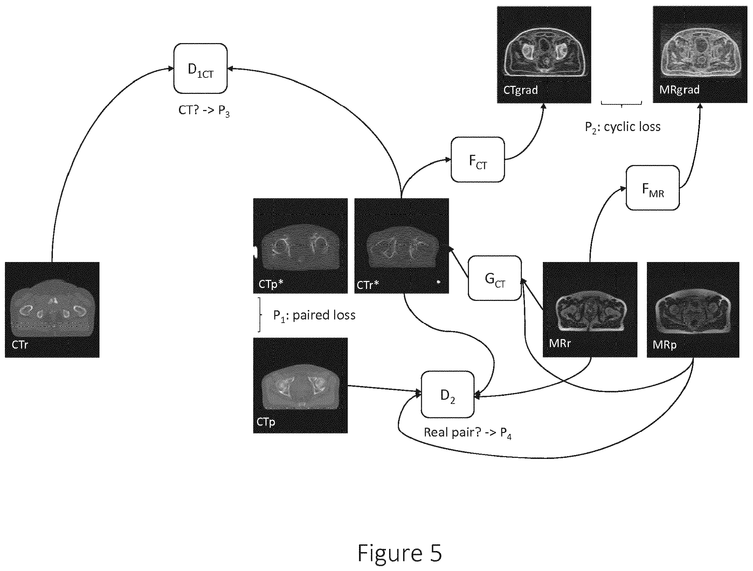

Figure 5 schematically illustrates a calculation of a second penalty P₂ in an embodiment where both the images used for calculating P₂ are converted into a third image type before they are compared. CTgrad in figure 5 corresponds to an original medical image MRr of a first image type (MR image) which has been converted into a converted medical image CTr* of a second image type (CT image) using a conversion function G_{CT}, and then converted again, into an image CTgrad of a third image type, in this example a gradient image, using another conversion function F_{CT}. MRgrad in figure 5 corresponds to the same original medical image MRr of the first image type (MR image) which has been converted into an image MRgrad of the same third image type, in this example a gradient image, using a conversion function F_{MR}. In the example shown in figure 5, the conversion functions F_{CT} and F_{MR} is the same function, but the conversion functions F_{CT} and F_{MR} may also be different functions.

The at least one processing unit 120 may e.g. obtain the initial parametrized conversion function G by generating it using a machine learning algorithm such as a neural network, a random forest, or a support vector machine. The machine learning algorithm may e.g. be a Generative Adversarial Network (GAN). This is a useful way of obtaining an initial parametrized conversion function G that may then be optimized by the system.

The at least one processing unit 120 may in further iterations obtain the initial parametrized conversion function G by using a previously generated optimized parametrized conversion function T as the initial parametrized conversion function G. This allows for iterative optimization of the parametrized conversion function T.

The first parametrized conversion function G₁ and/or the second parametrized conversion function G₂ may e.g. be the same as the initial parametrized conversion function G. They may however also be different, e.g. by the first parametrized conversion function G₁ being based on the parameters of the initial parametrized conversion function G and the first penalty P₁, and the second parametrized conversion function G₂ being based on the parameters of either the initial parametrized conversion function G or the first parametrized conversion function G₁ and the second penalty P₂. The same may apply to the third and the fourth parametrized conversion functions G₃ and G₄. If the penalties are applied one after the other, the parametrized conversion function adapted based on one penalty may thus e.g. be used when calculating the subsequent penalty.

The image type may e.g. be a medical image modality, such as e.g. MR, CT or CBCT. In other embodiments, the image type is the output from a pre-trained machine learning algorithm such as a convolutional neural network. In other embodiments, the medical images are converted into a third image type by being segmented, by being transferred into another domain by e.g. the gradient or the frequency content in the images being calculated, or by the resolution the images being changed. In other embodiments, the first image type is a medical image of a certain medical image modality, and the second image type is an image showing the density, the stopping power, the material composition, and/or any other feature that may be used for dose calculation. In other embodiments, the first image type is a segmented image and the second image type is a medical image of a certain medical image modality. This may be useful e.g. for creating hypothetical patient images in order to simulate radiation treatment based on such hypothetical patient images.

In one or more embodiments, the system 100 may optionally comprise a display 140 configured to display images. The display 140 may be configured to receive images for display via the at least one processing unit 120, and/or to retrieve images for display directly from the storage media 110, possibly in response to a control signal received from the at least one processing unit 120 or an inputter 150, which is further presented below.

In some embodiments, the system 100 may further optionally comprise one or more inputters 150 configured to receive user input. The inputter 150 is typically configured to interpret received user input and to generate control signals in response to said received user input. The display 140 and the inputter 150 may be integrated in, connected to or communicatively coupled to the system 100. The inputter 150 may for instance be configured to interpret received user input that is being input in connection with a displayed image, and generate control signals in response to said received user input, to trigger display of an image or manipulation of images being displayed, wherein the manipulations may be temporary or permanent. Such manipulations may for example include providing annotations, moving or changing an image or part of an image, changing the viewing perspective, zooming in or out, and/or any other suitable form of manipulation that enables the user to view and analyze the displayed image data in an improved manner. An inputter 150 may for example comprise a selection of a keyboard, a computer mouse, one or more buttons, touch functionality, a joystick, and/or any other suitable input device. In some embodiments, the processor 120 may be configured to receive a control signal from the inputter 150 and to process an image that is being displayed, or in other words manipulate a displayed image, in response to the received control signal.

The at least one processing unit 120 may further be configured to perform the method steps of any or all of the embodiments presented herein.

### Method embodiments

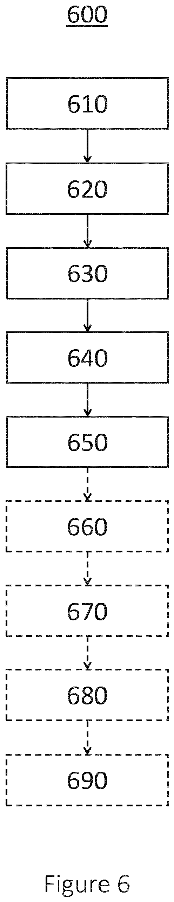

Figure 6 schematically illustrates a processor-implemented method of generating an optimized parametrized conversion function T for converting an original medical image of a first image type into a converted medical image of a second image type, in accordance with one or more embodiments described herein. The method 600 may comprise:
Step 610: obtaining original medical images of the first and second image types.
Step 620: obtaining an initial parametrized conversion function G to convert original medical images of the first image type into converted medical images of the second image type.
Step 630: calculating a first penalty P₁ based on at least one comparison of a first original medical image of the second image type with a first converted medical image of the second image type, that has been generated by applying a first parametrized conversion function G₁, which is based on the initial parametrized conversion function G, to a first original medical image of the first image type that forms an image pair with the first original medical image of the second image type and has thereby been determined to show the same part of the same patient.
Step 640: calculating a second penalty P₂ based on at least one comparison of an original medical image of the first image type and a converted medical image of the second image type that has been generated by applying a second parametrized conversion function G₂, which is based on the initial parametrized conversion function G, to the original medical image of the first image type, after converting the original medical image of the first image type and/or the converted medical image of the second image type into images of the same image type.
Step 650: generating the optimized parametrized conversion function T based on the parameters of the initial parametrized conversion function G and at least said first and second penalties P₁ and P₂.

The initial parametrized conversion function G may e.g. be obtained 620 by being generated using a machine learning algorithm such as a neural network, a random forest, or a support vector machine. The machine learning algorithm may e.g. be a Generative Adversarial Network (GAN). This is a useful way of obtaining 620 an initial parametrized conversion function G that may then be optimized.

In further iterations, the initial parametrized conversion function G may be obtained 620 by a previously generated optimized parametrized conversion function T being used as the initial parametrized conversion function G. This allows for iterative optimization of the parametrized conversion function T.

The comparing of the first original medical image with the corresponding first converted medical image may e.g. voxel wise compare the images. Before the comparison, the original medical image of the first image type may be converted into the second image type, the converted medical image of the second image type may be converted into the first image type, or both images may be converted into a third image type. The third image type may e.g. amplify certain features such as contours, regions-of-interest (ROI) and/or structures in the images so that they may more easily be compared. The third image type may e.g. be a gradient image, a segmented image or the output from a pre-trained machine learning algorithm such as a convolutional neural network.

The first parametrized conversion function G₁ and/or the second parametrized conversion function G₂ may e.g. be the same as the initial parametrized conversion function G. They may however also be different, e.g. by the first parametrized conversion function G₁ being based on the parameters of the initial parametrized conversion function G and the first penalty P₁, and the second parametrized conversion function G₂ being based on the parameters of either the initial parametrized conversion function G or the first parametrized conversion function G₁ and the second penalty P₂. The same may apply to the third and the fourth parametrized conversion functions G₃ and G₄. If the penalties are applied one after the other, the parametrized conversion function adapted based on one penalty may thus e.g. be used when calculating the subsequent penalty.

The image type may e.g. be a medical image modality, such as e.g. MR, CT or CBCT. In other embodiments, the image type is the output from a pre-trained machine learning algorithm such as a convolutional neural network. In other embodiments, the medical images are converted into a third image type by being segmented, by being transferred into another domain by e.g. the gradient or the frequency content in the images being calculated, or by the resolution the images being changed. In other embodiments, the first image type is a medical image of a certain medical image modality, and the second image type is an image showing the density, the stopping power, the material composition, and/or any other feature that may be used for dose calculation. In other embodiments, the first image type is a segmented image and the second image type is a medical image of a certain medical image modality. This may be useful e.g. for creating hypothetical patient images in order to simulate radiation treatment based on such hypothetical patient images.

In embodiments, the method 600 further comprises:
Step 660: obtaining a first parametrized discriminator function D₁ to discriminate between original medical images of the second image type and converted medical images of the second image type.
Step 670: calculating a third penalty P₃ based on a classification error of the first parametrized discriminator function D₁ when trying to discriminate between at least one original medical image of the second image type and at least one converted medical image of the second image type that has been generated by applying a third parametrized conversion function G₃, which is based on the initial parametrized conversion function G, to at least one original medical image of the first image type.

The optimized parametrized conversion function T may in this case be generated 650 based on the parameters of the initial parametrized conversion function G and at least said first, second and third penalties P₁, P₂ and P₃.

The first parametrized discriminator function D₁ may e.g. be obtained 660 based on the parameters of the parametrized discriminator function D₁ used for generating said optimized parametrized conversion function T and at least said third penalty P₃, so that the first parametrized discriminator function D₁ is iteratively optimized. This further improves the accuracy of the optimized parametrized conversion function T.

In embodiments, the method 600 further comprises:
Step 680: obtaining a second parametrized discriminator function D₂ to discriminate between
   a) image pairs with corresponding original medical images, an original medical image of the first image type and an original medical image of the second image type, which have been paired and thereby determined to show the same part of the same patient; and
   b) image pairs of an original medical image of the first image type and a corresponding converted medical image of the second image type.
Step 690: calculating a fourth penalty P₄ based on a classification error of the second parametrized discriminator function D₂.

The parametrized conversion function T may in this case be generated 650 based on the parameters of the initial parametrized conversion function G and at least said first, second and fourth penalties P₁, P₂ and P₄.

The third and fourth penalties may of course also be combined, so that the parametrized conversion function T is generated based on the parameters of the initial parametrized conversion function G and at least said first, second, third and fourth penalties P₁, P₂, P₃ and P₄.

In embodiments, the penalties are combined in the form of a weighted sum of the penalties, so that the parameters are adapted e.g. based on the gradient of the weighted sum of the penalties. In other embodiments, the penalties are instead applied one after the other, so that the parameters are adapted based on one penalty at a time.

### Use case embodiment

To set the presently disclosed systems and methods in a larger context, the generating of an optimized parametrized conversion function T may in use case embodiments be preceded by the generating of original medical images.

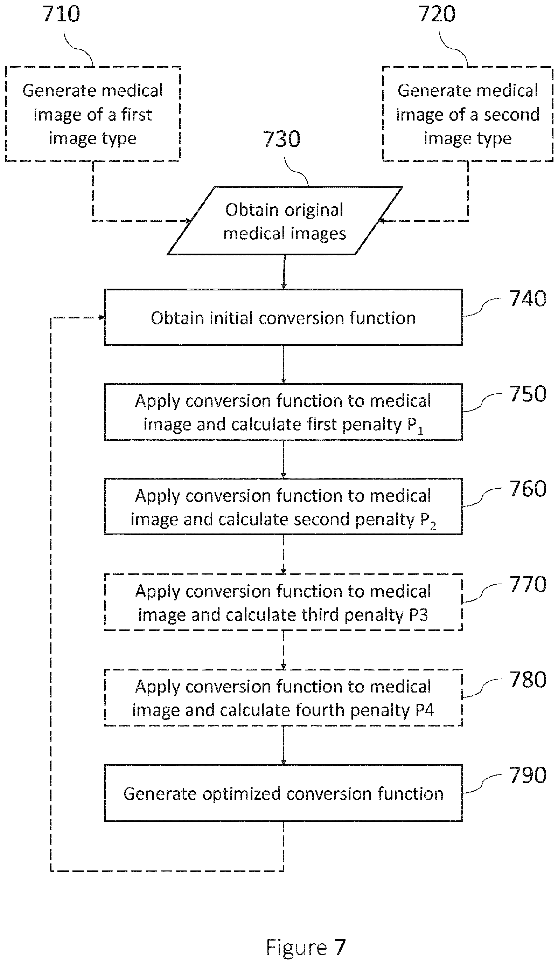

Figure 7 is a flow diagram exemplifying such a larger context, including the steps of: obtaining original medical images; obtaining an initial parametrized conversion function; calculating a first penalty P₁ based on paired images, by comparing the converted medical image of the second image type with the paired original medical image of the second image type; calculating a second penalty P₂ based on comparisons between converted medical images and original medical images, after converting them into images of the same image type; and generating an optimized parametrized conversion function based on the parameters of the initial parametrized conversion function and at least said first and second penalties P₁ and P₂. Figure 7 further includes steps of: generating the original medical images, e.g. by capturing them using image capturing systems such as magnetic resonance imaging (MR), computed tomography (CT), cone beam computed tomography (CBCT) and/or positron emission tomography (PET); calculating third and fourth penalties P₃ and P₄, and feeding back the conversion function for iteration. In figure 7, everything except the obtaining of original medical images, the obtaining of an initial parametrized conversion function, the calculating of a first penalty P₁, the calculating of a second penalty P₂, and optimizing the parametrized conversion function, is marked with dashed lines to clarify that they are optional steps shown in the figure to provide context only, and not essential to any of the embodiments presented herein. Especially, steps 710 and 720 relating to the generation of original medical images are not part of the embodiments presented herein.

According to the example shown in figure 7, an original medical image of a first image type may be generated in a step 710, e.g. by being captured by an image capturing system 130 using magnetic resonance imaging (MR), cone beam computed tomography (CBCT) and/or positron emission tomography (PET). An original medical image of a second image type may be generated in a step 720, e.g. by being captured using computed tomography (CT). These original medical images may be paired if they have been determined to show the same part of the same patient.

The original medical images may in step 730 be obtained either directly from the image capturing system 130 or from storage media 110. The initial parametrized conversion function G may in step 740 be obtained either by being determined by the at least one processing unit 120, e.g. using a machine learning algorithm, by being retrieved from storage media 110, or by feedback through using a previously generated parametrized conversion function T as the initial parametrized conversion function G, thereby iteratively optimizing the parametrized conversion function T.

In step 750, the initial parametrized conversion function G, or an adapted parametrized conversion function G₁, is applied to an original medical image of the first image type so that a converted medical image of the second image type is generated. This converted medical image is then compared to an original medical image of the second image type that forms an image pair with the original medical image of the first image type, so that it can be determined how closely the converted medical image of the second image type matches the original medical image of the second image type that shows the same the same part of the same patient. A first penalty P₁ is then calculated based on this comparison, which may e.g. be done voxel wise, and may e.g. compare features such as contours, regions-of-interest (ROI) and/or structures in the images, e.g. using a pre-trained machine learning algorithm such as a convolutional neural network.

In step 760, the initial parametrized conversion function G, or an adapted parametrized conversion function G₂, is applied to an original medical image of the first image type so that a converted medical image of the second image type is generated. The images are then converted into the same image type. This may be done by either converting one of the images into an image of the other image type, or converting both images into images of a third image type. In order to compare the images, they must however be of the same image type. The image type does not have to be a medical image modality. In other embodiments, the image type is the output from a pre-trained machine learning algorithm such as a convolutional neural network. In other embodiments, the medical images are converted into a third image type by being segmented, by being transferred into another domain by e.g. the gradient or the frequency content in the images being calculated, or by the resolution the images being changed. In other embodiments, the first image type is a medical image of a certain medical image modality, and the second image type is an image showing the density, the stopping power, the material composition, and/or any other feature that may be used for dose calculation. In other embodiments, the first image type is a segmented image and the second image type is a medical image of a certain medical image modality. What may be compared, e.g. voxel wise, may e.g. be features such as contours, regions-of-interest (ROI) and/or structures in the images, e.g. using a pre-trained machine learning algorithm such as a convolutional neural network. A second penalty P₂ is then calculated based on this comparison.

In step 770, which is an optional step, the initial parametrized conversion function G, or an adapted parametrized conversion function G₃, is applied to an original medical image of the first image type so that a converted medical image of the second image type is generated. A first discriminator function D₁ then tries to discriminate between the converted medical image of the second image type and an original medical image of the second image type. The original medical image used may be any original medical image, it does not have to be paired with the original medical image on which the converted medical image is based. A third penalty P₃ is then calculated based on a classification error of the first discriminator function D₁.

In step 780, which is also an optional step, the initial parametrized conversion function G, or an adapted parametrized conversion function G₄, is applied to an original medical image of the first image type so that a converted medical image of the second image type is generated. A second discriminator function D₂ then tries to discriminate between pairs of original paired medical images and pairs of original and corresponding converted medical images. A fourth penalty P₄ is then calculated based on a classification error of the discriminator function D₂.

In step 790, an optimized parametrized conversion function T is generated based on the parameters of the initial parametrized conversion function G and at least the first and second penalties P₁ and P₂, and optionally also the third penalty P₃ and/or the fourth penalty P₄. The penalties may be combined in the form of a weighted sum of the penalties, so that the parameters of the parametrized conversion function T are adapted based on the weighted sum of the penalties, or applied one after the other, so that the parameters of the parametrized conversion function T are adapted based on one penalty at a time.

In a use case embodiment, the optimized parametrized conversion function T may then be used for converting an original medical image of a first image type into a converted medical image of a second image type, which may e.g. be used for dose calculation in radiation treatment planning, where machine instructions e.g. in the form of a control signal may be sent to radiation treatment equipment based on an analysis of the converted medical image of the second image type.

### Further embodiments

Where applicable, various embodiments provided by the present disclosure can be implemented using hardware, software, or combinations of hardware and software. Also where applicable, the various hardware components and/or software components set forth herein can be combined into composite components comprising software, hardware, and/or both without departing from the claimed scope of the present disclosure. Where applicable, the various hardware components and/or software components set forth herein can be separated into sub-components comprising software, hardware, or both without departing from the claimed scope of the present disclosure. In addition, where applicable, it is contemplated that software components can be implemented as hardware components, and vice-versa. The method steps of one or more embodiments described herein may be performed automatically, by any suitable processing unit, or one or more steps may be performed manually. Where applicable, the ordering of various steps described herein can be changed, combined into composite steps, and/or separated into sub-steps to provide features described herein.

Software in accordance with the present disclosure, such as program code and/or data, can be stored in non-transitory form on one or more machine-readable mediums. It is also contemplated that software identified herein can be implemented using one or more general purpose or specific purpose computers and/or computer systems, networked and/or otherwise.

In embodiments, there are provided a computer program product comprising computer readable code configured to, when executed in a processor, perform any or all of the method steps described herein. In some embodiments, there are provided a non-transitory computer readable memory on which is stored computer readable and computer executable code configured to, when executed in a processor, perform any or all of the method steps described herein.

In one or more embodiments, there is provided a non-transitory machine-readable medium on which is stored machine-readable code which, when executed by a processor, controls the processor to perform the method of any or all of the method embodiments presented herein.

The foregoing disclosure is not intended to limit the present invention to the precise forms or particular fields of use disclosed. It is contemplated that various alternate embodiments and/or modifications to the present invention, whether explicitly described or implied herein, are possible in light of the disclosure. Accordingly, the scope of the invention is defined only by the claims.

## Claims

1. A system (100) for generating an optimized parametrized conversion function T for converting an original medical image of a first image type into a converted medical image of a second image type, the system comprising at least one processing unit configured to:
obtain original medical images of the first and second image types;
obtain an initial parametrized conversion function G to convert original medical images of the first image type into converted medical images of the second image type;
calculate a first penalty P₁ based on at least one comparison of a first original medical image of the second image type with a first converted medical image of the second image type, that has been generated by applying a first parametrized conversion function G₁, which is based on the initial parametrized conversion function G, to a first original medical image of the first image type that forms an image pair with the first original medical image of the second image type and has thereby been determined to show the same part of the same patient;
calculate a second penalty P₂ based on at least one comparison of an original medical image of the first image type and a converted medical image of the second image type that has been generated by applying a second parametrized conversion function G₂, which is based on the initial parametrized conversion function G, to the original medical image of the first image type, after converting the original medical image of the first image type and/or the converted medical image of the second image type into images of the same image type; and
generate the optimized parametrized conversion function T based on the parameters of the initial parametrized conversion function G and at least said first and second penalties P₁ and P₂.

2. A system according to claim 1, wherein the at least one processing unit is further configured to:
obtain a first parametrized discriminator function D₁ to discriminate between original medical images of the second image type and converted medical images of the second image type; and
calculate a third penalty P₃ based on a classification error of the first parametrized discriminator function D₁ when trying to discriminate between at least one original medical image of the second image type and at least one converted medical image of the second image type that has been generated by applying a third parametrized conversion function G₃, which is based on the initial parametrized conversion function G, to at least one original medical image of the first image type;
wherein the at least one processing unit generates the parametrized conversion function T based on the parameters of the initial parametrized conversion function G and at least said first, second and third penalties P₁, P₂ and P₃.

3. A system according to claim 1 or 2, wherein the at least one processing unit is further configured to:
obtain a second parametrized discriminator function D₂ to discriminate between
a) image pairs with corresponding original medical images, an original medical image of the first image type and an original medical image of the second image type, which have been paired and thereby determined to show the same part of the same patient; and
b) image pairs of an original medical image of the first image type and a corresponding converted medical image of the second image type; and
calculate a fourth penalty P₄ based on a classification error of the second parametrized discriminator function D₂;
wherein the at least one processing unit generates the parametrized conversion function T based on the parameters of the initial parametrized conversion function G and at least said first, second and fourth penalties P₁, P₂ and P₄.

4. A system according to any one of claims 1-3, wherein the at least one processing unit obtains the initial parametrized conversion function G by using a previously generated optimized parametrized conversion function T as the initial parametrized conversion function G, so that the parametrized conversion function T is iteratively optimized.

5. A processor-implemented method (600) of generating an optimized parametrized conversion function T for converting an original medical image of a first image type into a converted medical image of a second image type, the method comprising:
obtaining (610) original medical images of the first and second image types;
obtaining (620) an initial parametrized conversion function G to convert original medical images of the first image type into converted medical images of the second image type;
calculating (630) a first penalty P₁ based on at least one comparison of a first original medical image of the second image type with a first converted medical image of the second image type, that has been generated by applying a first parametrized conversion function G₁, which is based on the initial parametrized conversion function G, to a first original medical image of the first image type that forms an image pair with the first original medical image of the second image type and has thereby been determined to show the same part of the same patient;
calculating (640) a second penalty P₂ based on at least one comparison of an original medical image of the first image type and a converted medical image of the second image type that has been generated by applying a second parametrized conversion function G₂, which is based on the initial parametrized conversion function G, to the original medical image of the first image type, after converting the original medical image of the first image type and/or the converted medical image of the second image type into images of the same image type; and
generating (650) the optimized parametrized conversion function T based on the parameters of the initial parametrized conversion function G and at least said first and second penalties P₁ and P₂.

6. A method according to claim 5, further comprising:
obtaining (660) a first parametrized discriminator function D₁ to discriminate between original medical images of the second image type and converted medical images of the second image type; and
calculating (670) a third penalty P₃ based on a classification error of the first parametrized discriminator function D₁ when trying to discriminate between at least one original medical image of the second image type and at least one converted medical image of the second image type that has been generated by applying a third parametrized conversion function G₃, which is based on the initial parametrized conversion function G, to at least one original medical image of the first image type;
wherein the parametrized conversion function T is generated (640) based on the parameters of the initial parametrized conversion function G and at least said first, second and third penalties P₁, P₂ and P₃.

7. A method according to claim 6, further comprising obtaining (660) the first parametrized discriminator function D₁ based on the parameters of the parametrized discriminator function D₁ used for generating said optimized parametrized conversion function T and at least said third penalty P₃, so that the first parametrized discriminator function D₁ is iteratively optimized.

8. A method according to any one of claims 5-7, further comprising:
obtaining (680) a second parametrized discriminator function D₂ to discriminate between
a) image pairs with corresponding original medical images, an original medical image of the first image type and an original medical image of the second image type, which have been paired and thereby determined to show the same part of the same patient; and
b) image pairs of an original medical image of the first image type and a corresponding converted medical image of the second image type; and
calculating (690) a fourth penalty P₄ based on a classification error of the second parametrized discriminator function D₂;
wherein the parametrized conversion function T is generated (650) based on the parameters of the initial parametrized conversion function G and at least said first, second and fourth penalties P₁, P₂ and P₄.

9. A method according to any one of claims 5-8, further comprising obtaining (620) the initial parametrized conversion function G by generating it using a machine learning algorithm such as a neural network, a random forest, or a support vector machine.

10. A method according to any one of claims 5-8, wherein the initial parametrized conversion function G is obtained (620) by a previously generated optimized parametrized conversion function T being used as the initial parametrized conversion function G, so that the parametrized conversion function T is iteratively optimized.

11. A method according to any one of claims 5-10, wherein the comparing of the first original medical image with the corresponding first converted medical image voxel wise compares the images, after first converting both images into a third image type such as e.g. a gradient image, a segmented image, or the output from a pre-trained machine learning algorithm such as a convolutional neural network, wherein the third image type amplifies certain features such as contours, regions-of-interest (ROI) and/or structures in the images so that they may more easily be compared.

12. A method according to any one of claims 5-11, wherein the first parametrized conversion function G₁ and/or the second parametrized conversion function G₂ are the same as the initial parametrized conversion function G.

13. A method according to any one of claims 5-12, wherein the image type is a medical image modality, such as e.g. MR, CT or CBCT.

14. A system according to claim 2, wherein the at least one processing unit obtains the first parametrized discriminator function D₁ based on the parameters of the parametrized discriminator function D₁ used for generating said optimized parametrized conversion function T and at least said third penalty P₃, so that the first parametrized discriminator function D₁ is iteratively optimized.

15. A system according to any one of claims 1-3 or 14, wherein the at least one processing unit obtains the initial parametrized conversion function G by generating it using a machine learning algorithm such as a neural network, a random forest, or a support vector machine.

16. A system according to any one of claims 1-4, 14 or 15, wherein the at least one processing unit compares the first original medical image with the corresponding first converted medical image by voxel wise comparing the images, after first converting both images into a third image type such as e.g. a gradient image, a segmented image, or the output from a pre-trained machine learning algorithm such as a convolutional neural network, wherein the third image type amplifies certain features such as contours, regions-of-interest (ROI) and/or structures in the images so that they may more easily be compared.

17. A system according to any one of claims 1-4 or 14-16, wherein the first parametrized conversion function G₁ and/or the second parametrized conversion function G₂ are the same as the initial parametrized conversion function G.

18. A system according to any one of claims 1-4 or 14-17, wherein the image type is a medical image modality, such as e.g. MR, CT or CBCT.

## Patentansprüche

1. System (100) zum Erzeugen einer optimierten parametrisierten Umwandlungsfunktion T zum Umwandeln eines ursprünglichen medizinischen Bildes eines ersten Bildtyps in ein umgewandeltes medizinisches Bild eines zweiten Bildtyps, wobei das System mindestens eine Verarbeitungseinheit umfasst, die konfiguriert ist zum:
Erhalten von ursprünglichen medizinischen Bildern des ersten und des zweiten Bildtyps;
Erhalten einer anfänglichen parametrisierten Umwandlungsfunktion G zum Umwandeln ursprünglicher medizinischer Bilder des ersten Bildtyps in umgewandelte medizinische Bilder des zweiten Bildtyps;
Berechnen eines ersten Strafwerts P₁ basierend auf mindestens einem Vergleich eines ersten ursprünglichen medizinischen Bildes des zweiten Bildtyps mit einem ersten umgewandelten medizinischen Bild des zweiten Bildtyps, welches durch Anwenden einer ersten parametrisierten Umwandlungsfunktion G₁, die auf der anfänglichen parametrisierten Umwandlungsfunktion G basiert, auf ein erstes ursprüngliches medizinisches Bild des ersten Bildtyps erzeugt worden ist, welches ein Bildpaar mit dem ersten ursprünglichen medizinischen Bild des zweiten Bildtyps bildet und für welches dadurch bestimmt wurde, dass es denselben Teil desselben Patienten zeigt;
Berechnen eines zweiten Strafwerts P₂ basierend auf mindestens einem Vergleich eines ursprünglichen medizinischen Bildes des ersten Bildtyps und eines umgewandelten medizinischen Bildes des zweiten Bildtyps, welches durch Anwenden einer zweiten parametrisierten Umwandlungsfunktion G₂, die auf der anfänglichen parametrisierten Umwandlungsfunktion G basiert, auf das ursprüngliche medizinische Bild des ersten Bildtyps erzeugt worden ist, nach dem Umwandeln des ursprünglichen medizinischen Bildes des ersten Bildtyps und/oder des umgewandelten medizinischen Bildes des zweiten Bildtyps in Bilder desselben Bildtyps; und
Erzeugen der optimierten parametrisierten Umwandlungsfunktion T basierend auf den Parametern der anfänglichen parametrisierten Umwandlungsfunktion G und zumindest dem ersten und dem zweiten Strafwert P₁ und P₂.

2. System nach Anspruch 1, wobei die mindestens eine Verarbeitungseinheit ferner konfiguriert ist zum:
Erhalten einer ersten parametrisierten Diskriminatorfunktion D₁ zum Unterscheiden zwischen ursprünglichen medizinischen Bildern des zweiten Bildtyps und umgewandelten medizinischen Bildern des zweiten Bildtyps; und
Berechnen eines dritten Strafwerts P₃ basierend auf einem Klassifizierungsfehler der ersten parametrisierten Diskriminatorfunktion D₁, wenn versucht wird, zwischen mindestens einem ursprünglichen medizinischen Bild des zweiten Bildtyps und mindestens einem umgewandelten medizinischen Bild des zweiten Bildtyps zu unterscheiden, welches durch Anwenden einer dritten parametrisierten Umwandlungsfunktion G₃, die auf der anfänglichen parametrisierten Umwandlungsfunktion G basiert, auf mindestens ein ursprüngliches medizinisches Bild des ersten Bildtyps erzeugt worden ist;
wobei die mindestens eine Verarbeitungseinheit die parametrisierte Umwandlungsfunktion T basierend auf den Parametern der anfänglichen parametrisierten Umwandlungsfunktion G und zumindest auf dem ersten, dem zweiten und dem dritten Strafwert P₁, P₂ und P₃ erzeugt.

3. System nach Anspruch 1 oder 2, wobei die mindestens eine Verarbeitungseinheit ferner konfiguriert ist zum:
Erhalten einer zweiten parametrisierten Diskriminatorfunktion D₂ zum Unterscheiden zwischen
a) Bildpaaren mit entsprechenden ursprünglichen medizinischen Bildern, einem ursprünglichen medizinischen Bild des ersten Bildtyps und einem ursprünglichen medizinischen Bild des zweiten Bildtyps, die paarweise zusammengestellt und für welche dadurch bestimmt worden sind, dass sie denselben Teil desselben Patienten zeigen; und
b) Bildpaaren eines ursprünglichen medizinischen Bildes des ersten Bildtyps und eines entsprechenden umgewandelten medizinischen Bildes des zweiten Bildtyps; und
Berechnen eines vierten Strafwerts P₄ basierend auf einem Klassifizierungsfehler der zweiten parametrisierten Diskriminatorfunktion D₂;
wobei die mindestens eine Verarbeitungseinheit die parametrisierte Umwandlungsfunktion T basierend auf den Parametern der anfänglichen parametrisierten Umwandlungsfunktion G und zumindest auf dem ersten, dem zweiten und dem vierten Strafwert P₁, P₂ und P₄ erzeugt.

4. System nach einem der Ansprüche 1 bis 3, wobei die mindestens eine Verarbeitungseinheit die anfängliche parametrisierte Umwandlungsfunktion G unter Verwendung einer zuvor erzeugten optimierten parametrisierten Umwandlungsfunktion T als die anfängliche parametrisierte Umwandlungsfunktion G erhält, sodass die parametrisierte Umwandlungsfunktion T iterativ optimiert wird.

5. Prozessorimplementiertes Verfahren (600) zum Erzeugen einer optimierten parametrisierten Umwandlungsfunktion T zum Umwandeln eines ursprünglichen medizinischen Bildes eines ersten Bildtyps in ein umgewandeltes medizinisches Bild eines zweiten Bildtyps, wobei das Verfahren umfasst:
Erhalten (610) von ursprünglichen medizinischen Bildern des ersten und des zweiten Bildtyps;
Erhalten (620) einer anfänglichen parametrisierten Umwandlungsfunktion G zum Umwandeln ursprünglicher medizinischer Bilder des ersten Bildtyps in umgewandelte medizinische Bilder des zweiten Bildtyps;
Berechnen (630) eines ersten Strafwerts P₁ basierend auf mindestens einem Vergleich eines ersten ursprünglichen medizinischen Bildes des zweiten Bildtyps mit einem ersten umgewandelten medizinischen Bild des zweiten Bildtyps, welches durch Anwenden einer ersten parametrisierten Umwandlungsfunktion G₁, die auf der anfänglichen parametrisierten Umwandlungsfunktion G basiert, auf ein erstes ursprüngliches medizinisches Bild des ersten Bildtyps erzeugt worden ist, welches ein Bildpaar mit dem ersten ursprünglichen medizinischen Bild des zweiten Bildtyps bildet und für welches dadurch bestimmt wurde, dass es denselben Teil desselben Patienten zeigt;
Berechnen (640) eines zweiten Strafwerts P₂ basierend auf mindestens einem Vergleich eines ursprünglichen medizinischen Bildes des ersten Bildtyps und eines umgewandelten medizinischen Bildes des zweiten Bildtyps, welches durch Anwenden einer zweiten parametrisierten Umwandlungsfunktion G₂, die auf der anfänglichen parametrisierten Umwandlungsfunktion G basiert, auf das ursprüngliche medizinische Bild des ersten Bildtyps erzeugt worden ist, nach dem Umwandeln des ursprünglichen medizinischen Bildes des ersten Bildtyps und/oder des umgewandelten medizinischen Bildes des zweiten Bildtyps in Bilder desselben Bildtyps; und
Erzeugen (650) der optimierten parametrisierten Umwandlungsfunktion T basierend auf den Parametern der anfänglichen parametrisierten Umwandlungsfunktion G und zumindest dem ersten und dem zweiten Strafwert P₁ und P₂.

6. Verfahren nach Anspruch 5, ferner umfassend:
Erhalten (660) einer ersten parametrisierten Diskriminatorfunktion D₁ zum Unterscheiden zwischen ursprünglichen medizinischen Bildern des zweiten Bildtyps und umgewandelten medizinischen Bildern des zweiten Bildtyps; und
Berechnen (670) eines dritten Strafwerts P₃ basierend auf einem Klassifizierungsfehler der ersten parametrisierten Diskriminatorfunktion D₁, wenn versucht wird, zwischen mindestens einem ursprünglichen medizinischen Bild des zweiten Bildtyps und mindestens einem umgewandelten medizinischen Bild des zweiten Bildtyps zu unterscheiden, welches durch Anwenden einer dritten parametrisierten Umwandlungsfunktion G₃, die auf der anfänglichen parametrisierten Umwandlungsfunktion G basiert, auf mindestens ein ursprüngliches medizinisches Bild des ersten Bildtyps erzeugt worden ist;
wobei die parametrisierte Umwandlungsfunktion T basierend auf den Parametern der anfänglichen parametrisierten Umwandlungsfunktion G und zumindest auf dem ersten, dem zweiten und dem dritten Strafwert P₁, P₂ und P₃ erzeugt wird (640).

7. Verfahren nach Anspruch 6, ferner umfassend Erhalten (660) der ersten parametrisierten Diskriminatorfunktion D₁ basierend auf den Parametern der parametrisierten Diskriminatorfunktion D₁, die zum Erzeugen der optimierten parametrisierten Umwandlungsfunktion T verwendet wird, und zumindest dem dritten Strafwert P₃, sodass die erste parametrisierte Diskriminatorfunktion D₁ iterativ optimiert wird.

8. Verfahren nach einem der Ansprüche 5 bis 7, ferner umfassend:
Erhalten (680) einer zweiten parametrisierten Diskriminatorfunktion D₂ zum Unterscheiden zwischen
a) Bildpaaren mit entsprechenden ursprünglichen medizinischen Bildern, einem ursprünglichen medizinischen Bild des ersten Bildtyps und einem ursprünglichen medizinischen Bild des zweiten Bildtyps, die paarweise zusammengestellt und für welche dadurch bestimmt worden sind, dass sie denselben Teil desselben Patienten zeigen; und
b) Bildpaaren eines ursprünglichen medizinischen Bildes des ersten Bildtyps und eines entsprechenden umgewandelten medizinischen Bildes des zweiten Bildtyps; und
Berechnen (690) eines vierten Strafwerts P₄ basierend auf einem Klassifizierungsfehler der zweiten parametrisierten Diskriminatorfunktion D₂;
wobei die parametrisierte Umwandlungsfunktion T basierend auf den Parametern der anfänglichen parametrisierten Umwandlungsfunktion G und zumindest auf dem ersten, dem zweiten und dem vierten Strafwert P₁, P₂ und P₄ erzeugt wird (650).

9. Verfahren nach einem der Ansprüche 5 bis 8, ferner umfassend Erhalten (620) der anfänglichen parametrisierten Umwandlungsfunktion G durch Erzeugen derselben unter Verwendung eines Maschinenlernalgorithmus, wie eines neuronalen Netzes, eines Zufallswalds oder einer Support-Vektor-Maschine.

10. Verfahren nach einem der Ansprüche 5 bis 8, wobei die anfängliche parametrisierte Umwandlungsfunktion G erhalten wird (620), indem eine zuvor erzeugte optimierte parametrisierte Umwandlungsfunktion T als die anfängliche parametrisierte Umwandlungsfunktion G verwendet wird, sodass die parametrisierte Umwandlungsfunktion T iterativ optimiert wird.

11. Verfahren nach einem der Ansprüche 5 bis 10, wobei beim Vergleichen des ersten ursprünglichen medizinischen Bildes mit dem entsprechenden ersten umgewandelten medizinischen Bild die Bilder voxelweise verglichen werden, nachdem zuerst beide Bilder in einen dritten Bildtyp umgewandelt wurden, wie ein Gradientenbild, ein segmentiertes Bild oder der Ausgabe von einem vorab trainierten Maschinenlernalgorithmus, wie einem neuronalen Faltungsnetz, wobei der dritte Bildtyp bestimmte Merkmale, wie Konturen, Regionen von Interesse (Regions of Interest, ROI) und/oder Strukturen in den Bildern verstärkt, sodass sie einfacher verglichen werden können.

12. Verfahren nach einem der Ansprüche 5 bis 11, wobei die erste parametrisierte Umwandlungsfunktion G₁ und/oder die zweite parametrisierte Umwandlungsfunktion G₂ die gleichen wie die anfängliche parametrisierte Umwandlungsfunktion G sind.

13. Verfahren nach einem der Ansprüche 5 bis 12, wobei der Bildtyp eine medizinische Bildmodalität ist, wie MR, CT oder CBCT.

14. System nach Anspruch 2, wobei die mindestens eine Verarbeitungseinheit die erste parametrisierte Diskriminatorfunktion D₁ basierend auf den Parametern der parametrisierten Diskriminatorfunktion D₁, die zum Erzeugen der optimierten parametrisierten Umwandlungsfunktion T verwendet wird, und zumindest auf dem dritten Strafwert P₃ erhält, sodass die erste parametrisierte Diskriminatorfunktion D₁ iterativ optimiert wird.

15. System nach einem der Ansprüche 1 bis 3 oder 14, wobei die mindestens eine Verarbeitungseinheit die anfängliche parametrisierte Umwandlungsfunktion G erhält, indem sie sie unter Verwendung eines Maschinenlernalgorithmus erzeugt, wie eines neuronalen Netzes, eines Zufallswalds oder einer Support-Vektor-Maschine.

16. System nach einem der Ansprüche 1 bis 4, 14 oder 15, wobei die mindestens eine Verarbeitungseinheit das erste ursprüngliche medizinische Bild mit dem entsprechenden ersten umgewandelten medizinischen Bild durch voxelweises Vergleichen der Bilder vergleicht, nachdem beide Bilder zuerst in einen dritten Bildtyp umgewandelt wurden, wie ein Gradientenbild, ein segmentiertes Bild oder die Ausgabe aus einem vorab trainierten Maschinenlernalgorithmus, beispielsweise einem neuronalen Faltungsnetz, wobei der dritte Bildtyp bestimmte Merkmale wie Konturen, Regionen von Interesse (ROI) und/oder Strukturen in den Bildern verstärkt, sodass sie einfacher verglichen werden können.

17. System nach einem der Ansprüche 1 bis 4 oder 14 bis 16, wobei die erste parametrisierte Umwandlungsfunktion G₁ und/oder die zweite parametrisierte Umwandlungsfunktion G₂ die gleichen wie die anfängliche parametrisierte Umwandlungsfunktion G sind.

18. System nach einem der Ansprüche 1 bis 4 oder 14 bis 17, wobei der Bildtyp eine medizinische Bildmodalität ist, wie MR, CT oder CBCT.

## Revendications

1. Système (100) pour générer une fonction de conversion paramétrée optimisée T pour convertir une image médicale originale d'un premier type d'image en une image médicale convertie d'un deuxième type d'image, le système comprenant au moins une unité de traitement configurée pour :
obtenir des images médicales originales des premier et deuxième types d'image ;
obtenir une fonction de conversion paramétrée initiale G pour convertir des images médicales originales du premier type d'image en images médicales converties du deuxième type d'image ;
calculer une première pénalité P₁ sur la base d'au moins une comparaison d'une première image médicale originale du deuxième type d'image avec une première image médicale convertie du deuxième type d'image, qui a été générée en appliquant une première fonction de conversion paramétrée G₁, qui est basée sur la fonction de conversion paramétrée initiale G, à une première image médicale originale du premier type d'image qui forme une paire d'images avec la première image médicale originale du deuxième type d'image et a de ce fait été déterminée comme montrant la même partie du même patient ;
calculer une deuxième pénalité P₂ sur la base d'au moins une comparaison d'une image médicale originale du premier type d'image et d'une image médicale convertie du deuxième type d'image qui a été générée en appliquant une deuxième fonction de conversion paramétrée G₂, qui est basée sur la fonction de conversion paramétrée initiale G, à l'image médicale originale du premier type d'image, après conversion de l'image médicale originale du premier type d'image et/ou de l'image médicale convertie du deuxième type d'image en images du même type d'image ; et
générer la fonction de conversion paramétrée optimisée T sur la base des paramètres de la fonction de conversion paramétrée initiale G et au moins desdites première et deuxième pénalités P₁ et P₂.

2. Système selon la revendication 1, dans lequel l'au moins une unité de traitement est en outre configurée pour :
obtenir une première fonction discriminative paramétrée D₁ pour discriminer entre des images médicales originales du deuxième type d'image et des images médicales converties du deuxième type d'image ; et
calculer une troisième pénalité P₃ sur la base d'une erreur de classification de la première fonction discriminative paramétrée D₁ lors de la tentative de discrimination entre au moins une image médicale originale du deuxième type d'image et au moins une image médicale convertie du deuxième type d'image qui a été générée en appliquant une troisième fonction de conversion paramétrée G₃, qui est basée sur la fonction de conversion paramétrée initiale G, à au moins une image médicale originale du premier type d'image ;
dans lequel l'au moins une unité de traitement génère la fonction de conversion paramétrée T sur la base des paramètres de la fonction de conversion paramétrée initiale G et au moins desdites première, deuxième et troisième pénalités P₁, P₂ et P₃.

3. Système selon les revendications 1 ou 2, dans lequel l'au moins une unité de traitement est en outre configurée pour :
obtenir une deuxième fonction discriminative paramétrée D₂ pour discriminer entre
a) des paires d'images avec des images médicales originales correspondantes, une image médicale originale du premier type d'image et une image médicale originale du deuxième type d'image, qui ont été appariées et de ce fait déterminées comme montrant la même partie du même patient ; et
b) des paires d'images d'une image médicale originale du premier type d'image et d'une image médicale convertie correspondante du deuxième type d'image ; et
calculer une quatrième pénalité P₄ sur la base d'une erreur de classification de la deuxième fonction discriminative paramétrée D₂ ;
dans lequel l'au moins une unité de traitement génère la fonction de conversion paramétrée T sur la base des paramètres de la fonction de conversion paramétrée initiale G et au moins desdites première, deuxième et quatrième pénalités P₁, P₂ et P₄.

4. Système selon l'une quelconque des revendications 1 à 3, dans lequel l'au moins une unité de traitement obtient la fonction de conversion paramétrée initiale G en utilisant une fonction de conversion paramétrée optimisée T précédemment générée comme la fonction de conversion paramétrée initiale G, de sorte que la fonction de conversion paramétrée T soit optimisée de manière itérative.

5. Procédé mis en oeuvre par processeur (600) de génération d'une fonction de conversion paramétrée optimisée T pour convertir une image médicale originale d'un premier type d'image en une image médicale convertie d'un deuxième type d'image, le procédé comprenant :
l'obtention (610) d'images médicales originales des premier et deuxième types d'image ;
l'obtention (620) d'une fonction de conversion paramétrée initiale G pour convertir des images médicales originales du premier type d'image en images médicales converties du deuxième type d'image ;
le calcul (630) d'une première pénalité P₁ sur la base d'au moins une comparaison d'une première image médicale originale du deuxième type d'image avec une première image médicale convertie du deuxième type d'image, qui a été générée en appliquant une première fonction de conversion paramétrée G₁, qui est basée sur la fonction de conversion paramétrée initiale G, à une première image médicale originale du premier type d'image qui forme une paire d'images avec la première image médicale originale du deuxième type d'image et a de ce fait été déterminée comme montrant la même partie du même patient ;
le calcul (640) d'une deuxième pénalité P₂ sur la base d'au moins une comparaison d'une image médicale originale du premier type d'image et d'une image médicale convertie du deuxième type d'image qui a été générée en appliquant une deuxième fonction de conversion paramétrée G₂, qui est basée sur la fonction de conversion paramétrée initiale G, à l'image médicale originale du premier type d'image, après conversion de l'image médicale originale du premier type d'image et/ou de l'image médicale convertie du deuxième type d'image en images du même type d'image ; et
la génération (650) de la fonction de conversion paramétrée optimisée T sur la base des paramètres de la fonction de conversion paramétrée initiale G et au moins desdites première et deuxième pénalités P₁ et P₂.

6. Procédé selon la revendication 5, comprenant en outre :
l'obtention (660) d'une première fonction discriminative paramétrée D₁ pour discriminer entre des images médicales originales du deuxième type d'image et des images médicales converties du deuxième type d'image ; et
le calcul (670) d'une troisième pénalité P₃ sur la base d'une erreur de classification de la première fonction discriminative paramétrée D₁ lors de la tentative de discrimination entre au moins une image médicale originale du deuxième type d'image et au moins une image médicale convertie du deuxième type d'image qui a été générée en appliquant une troisième fonction de conversion paramétrée G₃, qui est basée sur la fonction de conversion paramétrée initiale G, à au moins une image médicale originale du premier type d'image ;
dans lequel la fonction de conversion paramétrée T est générée (640) sur la base des paramètres de la fonction de conversion paramétrée initiale G et au moins desdites première, deuxième et troisième pénalités P₁, P₂ et P₃.

7. Procédé selon la revendication 6, comprenant en outre l'obtention (660) de la première fonction discriminative paramétrée D₁ sur la base des paramètres de la fonction discriminative paramétrée D₁ utilisée pour générer ladite fonction de conversion paramétrée optimisée T et au moins ladite troisième pénalité P₃, de sorte que la première fonction discriminative paramétrée D₁ soit optimisée de manière itérative.

8. Procédé selon l'une quelconque des revendications 5 à 7, comprenant en outre :
l'obtention (680) d'une deuxième fonction discriminative paramétrée D₂ pour discriminer entre
a) des paires d'images avec des images médicales originales correspondantes, une image médicale originale du premier type d'image et une image médicale originale du deuxième type d'image, qui ont été appariées et de ce fait déterminées comme montrant la même partie du même patient ; et
b) des paires d'images d'une image médicale originale du premier type d'image et d'une image médicale convertie correspondante du deuxième type d'image ; et
calculer (690) une quatrième pénalité P₄ sur la base d'une erreur de classification de la deuxième fonction discriminative paramétrée D₂ ;
dans lequel la fonction de conversion paramétrée T est générée (650) sur la base des paramètres de la fonction de conversion paramétrée initiale G et au moins desdites première, deuxième et quatrième pénalités P₁, P₂ et P₄.

9. Procédé selon l'une quelconque des revendications 5 à 8, comprenant en outre l'obtention (620) de la fonction de conversion paramétrée initiale G en la générant en utilisant un algorithme d'apprentissage automatique tel qu'un réseau neuronal, une forêt aléatoire, ou une machine à vecteurs de support.

10. Procédé selon l'une quelconque des revendications 5 à 8, dans lequel la fonction de conversion paramétrée initiale G est obtenue (620) par une fonction de conversion paramétrée optimisée T précédemment générée étant utilisée comme la fonction de conversion paramétrée initiale G, de sorte que la fonction de conversion paramétrée T soit optimisée de manière itérative.

11. Procédé selon l'une quelconque des revendications 5 à 10, dans lequel la comparaison de la première image médicale originale avec la première image médicale convertie correspondante compare les images voxel par voxel, après première conversion des deux images en un troisième type d'image tel que par exemple une image-gradient, une image segmentée, ou le résultat d'un algorithme d'apprentissage automatique pré-entraîné tel qu'un réseau neuronal convolutif, dans lequel le troisième type d'image amplifie certains éléments tels que des contours, des régions d'intérêts (ROI) et/ou des structures dans les images de sorte qu'elles puissent être plus facilement comparées.

12. Procédé selon l'une quelconque des revendications 5 à 11, dans lequel la première fonction de conversion paramétrée G₁ et/ou la deuxième fonction de conversion paramétrée G₂ sont les mêmes que la fonction de conversion paramétrée initiale G.

13. Procédé selon l'une quelconque des revendications 5 à 12, dans lequel le type d'image est une modalité d'image médicale, telle que par exemple MR, CT ou CBCT.

14. Système selon la revendication 2, dans lequel l'au moins une unité de traitement obtient la première fonction discriminative paramétrée D₁ sur la base des paramètres de la fonction discriminative paramétrée D₁ utilisée pour générer ladite fonction de conversion paramétrée optimisée T et au moins ladite troisième pénalité P₃, de sorte que la première fonction discriminative paramétrée D₁ soit optimisée de manière itérative.

15. Système selon l'une quelconque des revendications 1 à 3 ou 14, dans lequel l'au moins une unité de traitement obtient la fonction de conversion paramétrée initiale G en la générant en utilisant un algorithme d'apprentissage automatique tel qu'un réseau neuronal, une forêt aléatoire, ou une machine à vecteurs de support.

16. Système selon l'une quelconque des revendications 1 à 4, 14 ou 15, dans lequel l'au moins une unité de traitement compare la première image médicale originale avec la première image médicale convertie correspondante en comparant les images voxel par voxel, après première conversion des deux images en un troisième type d'image tel que par exemple une image-gradient, une image segmentée, ou le résultat d'un algorithme d'apprentissage automatique pré-entraîné tel qu'un réseau neuronal convolutif, dans lequel le troisième type d'image amplifie certains éléments tels que des contours, des régions d'intérêts (ROI) et/ou des structures dans les images de sorte qu'elles puissent être plus facilement comparées.

17. Système selon l'une quelconque des revendications 1 à 4 ou 14 à 16, dans lequel la première fonction de conversion paramétrée G₁ et/ou la deuxième fonction de conversion paramétrée G₂ sont les mêmes que la fonction de conversion paramétrée initiale G.

18. Système selon l'une quelconque des revendications 1 à 4 ou 14 à 17, dans lequel le type d'image est une modalité d'image médicale, telle que par exemple MR, CT ou CBCT.
